# EUROPEAN PATENT APPLICATION

(11) **EP 4 015 012 A1**
(43) Date of publication of application: **22.06.2022**
(21) Application number: 21202695.9
(22) Date of filing: 14.10.2021
(51) Int. Cl.: A61M 1/06

(54) **A BREAST PUMP**

(30) Priority: 16.12.2020 EP 20214568
(71) Applicant: Koninklijke Philips N.V., 5656 AG Eindhoven (NL)
(72) Inventor: BOURQUIN, Yannyk Parulian Julian, Eindhoven (NL)
(74) Representative: Philips Intellectual Property & Standards

(57) **Abstract**

A breast pump comprises a breast shield for fitting over at least the nipple of a breast thereby to create a cavity over the breast. A pump is used to deliver an under pressure to the cavity. In addition, a compressible chamber is used to manually create a reduced pressure in the cavity, in particular to function as a baseline pressure for holding the breast shield against the breast.

## Description

### FIELD OF THE INVENTION

This invention relates to breast pumps.

### BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. During the stimulation mode, the milk ejection reflex is stimulated. When the breast pump is activated, a pressure source such as a vacuum pump inside the breast pump generates a vacuum and the stimulation mode can be started. The vacuum pump is typically driven by an electric motor. For portable breast pumps, these motors are driven by battery power.

There are also breast pumps using manually driven mechanical pumps.

Wearable breast pumps are characterized in that they can be fully worn on the user's body, allowing the user to move around freely. Some of these are small enough to fit into a user's bra. A motor, battery and milk container are for example integrated to form a single unit.

In order to avoid contamination of the pump with milk residue, many breast pumps employ a moveable membrane, known as a diaphragm, between the pump and the expression kit, the membrane forming a barrier against any milk being sucked into the pump. In some breast pumps, the membrane acts furthermore as a massaging element on the nipple, areola and/or breast of the user. The membrane touches parts of the user's breast during the use of the breast pump. There is movement away from the breast when vacuum is applied and towards the breast when vacuum is released. This stimulates the milk release of the user.

The operation of the breast pumps makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

By generating a pressure cycle, particularly a vacuum cycle, a simulation of a feeding action is obtained, which triggers the necessary let-down reflex in the lactating. However, a return to ambient pressure within the breast shield chamber may not be required, and benefits may be achieved by maintaining a minimum level of vacuum on the breast throughout at least a portion of the pumping session.

With the recent trend towards wearable breast pumps, there is a need for a pump that can maintain itself to the breast without the need of the user to hold it with the hand. The main issues that are encountered are the backflow of milk and the loss of vacuum. To solve this, a baseline vacuum is one of the solutions.

WO 2008/127991 A1 for example discloses a breast pump that includes a mechanism to regulate a pressure change within a breast shield chamber, including in some cases to a maintained minimum baseline pressure that is less than ambient (atmosphere). This however requires a complex mechanism to maintain the baseline vacuum in the breast shield. In particular, a pressure difference needs to be maintained between the breast shield and the container in order to ensure the milk flow can take place to the container. A complex arrangement of valves and pressure control results.

There remains a need for a simple way to provide baseline under pressure in the breast pump for holding the breast pump to the breast in a hands-free manner.

### SUMMARY OF THE INVENTION

The invention is defined by the claims.

According to examples in accordance with an aspect of the invention, there is provided a breast pump, comprising:
a breast shield for fitting over at least the nipple of a breast thereby to create a cavity over the breast;
a pump for delivering an under pressure to the cavity; and
a compressible chamber for manually creating a reduced pressure in the cavity.

It is noted that the pressure may be delivered to the cavity directly or indirectly by means of a membrane.

This breast pump design delivers a pressure for application to the cavity using a pump as well as an additional manually-generated reduced pressure, which in particular can function as a baseline pressure for holding the breast pump (i.e. the breast shield) against the breast of the user. This provides a simple solution for generating a holding pressure.

The pump is preferably an electric pump. The breast pump thus design uses an electrically generated pressure waveform for application to the cavity.

The compressible chamber is preferably elastic and delivers a restoring force to return to a non-compressed state. Thus, when compressed, it is the restoring force of the chamber that creates a pressure reduction in the cavity.

In one set of examples, the compressible chamber comprises a milk collection container. In this case, no significant additional parts are needed. Instead, the milk collection container has the two functions of generating a baseline under pressure as well as collecting the expressed milk.

The milk collection container for example has a volume greater than 120ml. This is greater than the volume typically needed for milk collection so that an additional volume serves the function of delivering under pressure. Thus, even when all milk has been expressed, the milk collection chamber remains partially compressed and continues to provide a restoring force.

In another set of examples, the breast pump further comprises a milk collection container, additional to the compressible chamber. In this case, the compressible chamber is used only to create a mechanical under pressure.

The breast pump preferably further comprises a first, one-way, valve between the breast shield and the milk collection container.

This valve allows milk to flow to the milk collection chamber. The first valve is for example a duckbill valve.

The breast pump preferably further comprises a second, one-way, valve from the compressible chamber to ambient surroundings. When the compressible chamber is compressed air is expelled through the second valve. When the compressible chamber tries to restore its shape, this second valve is closed (because the increase in volume of the compressible chamber creates a pressure drop) and the under pressure in the compressible chamber is thereby retained.

The breast shield preferably comprises a diaphragm between the cavity and the pump. This isolates the pump from the cavity. However it also means the pump can return to atmospheric pressure but an under pressure can be maintained in the cavity.

The compressible chamber for example generates a negative pressure between 2kPa and 10kPa (i.e. this amount below atmospheric pressure of around 100kPa).

The compressible chamber is for example formed of a rubber material such as silicone.

The breast pump for example comprises a wearable breast pump.

The invention also provides a method of operating a breast pump for the non-therapeutic expression of milk, comprising:
fitting a breast shield over at least the nipple of a breast thereby to create a cavity over the breast;
manually creating a reduced pressure in the cavity by compressing a compressible chamber;
delivering an under pressure to the cavity using a pump; and
collecting milk in a milk collection container.

The compressible chamber may comprise the milk collection container or else the compressible chamber may be additional to the milk collection container.

The method for example comprises delivering the under pressure using an electric pump.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a known breast pump system;
Figure 2 shows a wearable breast pump;
Figure 3 shows one example of a wearable breast pump in accordance with the invention;
Figure 4 shows the compressible chamber of the breast pump of Figure 3 being compressed manually; and
Figure 5 shows the cavity pressure over time.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump which comprises a breast shield for fitting over at least the nipple of a breast thereby to create a cavity over the breast. A pump is used to deliver an under pressure to the cavity. In addition, a compressible chamber is used to manually create a reduced pressure in the cavity, in particular to function as a baseline pressure for holding the breast shield against the breast.

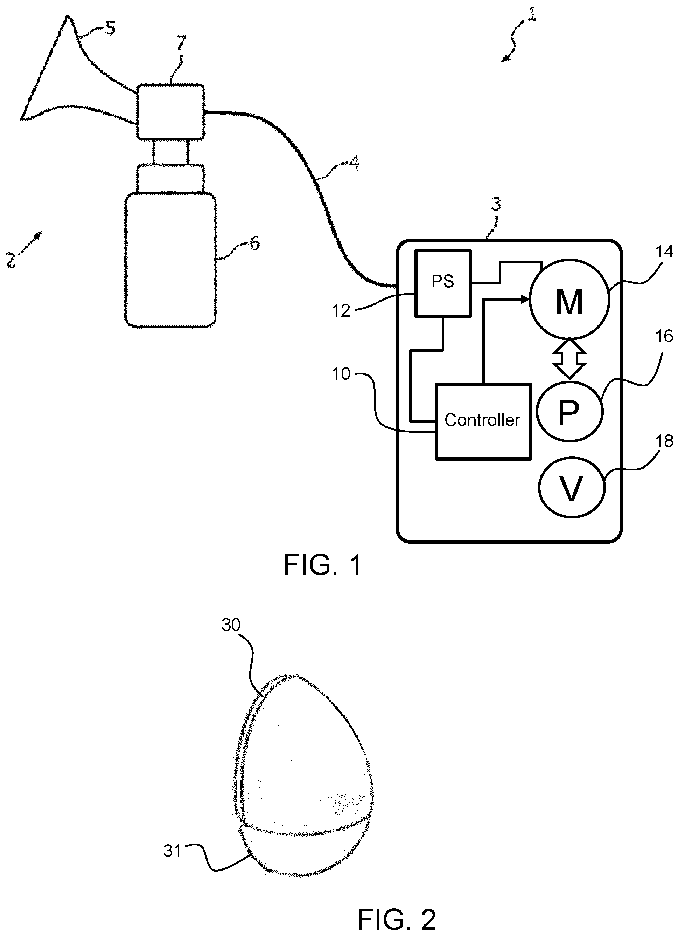

Figure 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the membrane prevents expressed milk from flowing directly into the pump arrangement 3.

The pump arrangement 3 comprises a controller 10, a power source 12, a motor 14 and a vacuum pump 16 (i.e. the impeller driven by the motor 14). The controller controls 10 the operation of the power source 12, motor 14 and vacuum pump 16. The pump arrangement 3 further comprises a solenoid valve 18.

In use, the vacuum pump 16 applies a vacuum to the membrane located in the main body 7 so that it deforms. The membrane deforms to create a vacuum in the funnel 5 which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely.

There are also other types of wearable breast pump whereby the expression unit is fixed to a breast and the pump unit and/or milk container are situated elsewhere on the body of a user.

Figure 2 shows a wearable breast pump, comprising an expression kit 30 attached to a respective milk collection container 31.

This invention relates to the use of a baseline pressure to hold the breast shield 5 against the breast. The invention is of particular interest for a wearable breast pump, but it may be applied to other types of breast pump as outlined above to improve ease of use.

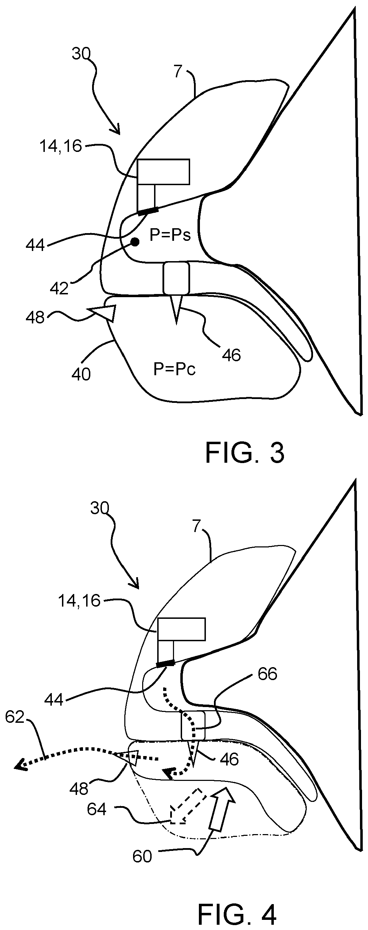

Figure 3 shows a wearable breast pump comprising an expression kit 30 as mentioned above and a milk collection container. However, instead of using a rigid milk collection container, in this example the milk collection container is in the form of a compressible chamber 40 for manually creating a reduced pressure in the pressure-controlled cavity of the breast pump.

The breast pump thus comprises a breast shield 7, for fitting over at least the nipple of a breast thereby to create a cavity 42 over the breast. The under-pressure in the cavity is delivered by a pump 14,16 which in this example is an electric pump. This under pressure is not directly delivered by the electric pump, but there is a membrane 44 between the pump and the cavity 42 over the nipple and breast.

The compressible chamber 40 is elastic so that it delivers a restoring force to return to a non-compressed state. Thus, when compressed, a restoring force is established, and this creates a pressure reduction in the collapsible chamber 40 and in turn in the cavity 42. The compressible chamber 40 is for example formed of a rubber material such as silicone.

A first, one-way, valve 46 is provided between the breast shield and the milk collection container. This first valve 46 allows milk to flow from the cavity 42 to the milk collection container. The first valve 46 is for example a duckbill valve. This valve also allows an under pressure to be transferred to the cavity 42 by suction from the compressible chamber 40. Thus, if the pressure in the cavity 42 is higher than in the compressible chamber 40, the valve will open and equalize the pressures.

The breast pump comprises a second, one-way, valve 48 from the compressible chamber 40 to ambient surroundings.

Figure 4 shows the compressible chamber being compressed manually, i.e. by the user performing a squeezing action represented by arrow 60. When the compressible chamber is compressed, air is expelled through the second valve 48 as shown by arrow 62. When the compressible chamber tries to restore its shape by the restoring force, represented by arrow 64, this second valve 48 is closed (because the increase in volume in the compressible chamber 40 creates a pressure drop). The under pressure in the compressible chamber 40 is thereby retained and it equalizes with the cavity as shown by arrow 66. This manually generated reduced pressure functions as a baseline pressure for holding the breast pump against the breast of the user. This provides a simple solution for generating a holding pressure.

Before applying the breast pump to the breast, the pressure in the compressible chamber (chamber pressure Pc) is equal to the pressure in the cavity (the breast shield pressure Ps) and equal to atmospheric pressure (Ps=Pc=atmospheric pressure).

When applying the device after squeezing the compressible chamber 40, the chamber pressure Pc and the shield pressure Ps drop such that Pc = Ps < Atm.

The air from the compressible chamber 40 container escapes from the second valve 48. The breast pump is then attached to the breast. When activating the pump to create an oscillating negative pressure waveform, the breast shield pressure Ps oscillates, whereas the chamber pressure Pc remains constant. The first valve 46 automatically closes when Ps<Pc. Thus, the oscillating negative pressure only occurs in the breast shield.

The user can at any time re-squeeze the compressible chamber 40 to readjust the level of negative pressure. In doing so, the air escapes only from the first valve 48 and does not enter the cavity through the first valve 46.

When using a standard pump, as is present in most current breast pumps, the vacuum profile within the breast shield will oscillate from a maximum negative pressure to the pressure Pc in the compressible chamber and will not reach atmospheric pressure. As a result, no modification is need to a standard pump design and control.

The difference in operation is that when the pump returns to atmospheric pressure, the pressure at the pump remains higher than at the breast shield. The membrane 44 between the pump and the breast shield cavity means the pump can return to atmospheric pressure while the breast shield cavity pressure Ps remains lower than atmospheric pressure as a result of the valves 46, 48 which remain closed.

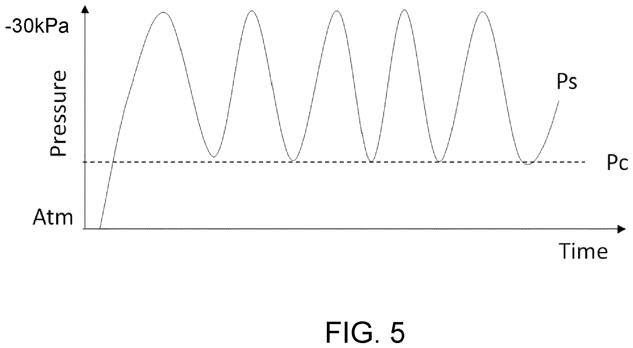

Figure 5 shows the cavity pressure over time. As shown, a cyclic pressure waveform is again created, in the same way as for a standard breast pump as explained above. The compressible chamber 40 contributes a constant baseline pressure Pc and the electrically generated oscillating waveform Ps is provided in addition. Note that the y-axis represents negative pressure, i.e. the amount by which the pressure is below the atmospheric pressure. The baseline pressure is for example in the range 2kPa to 10kPa, whereas the maximum under-pressure is around 30kPa (hence a pressure of -30kPa in Figure 5).

In the example shown, the compressible chamber 40 is the milk collection container. Thus, no significant additional parts are needed. Instead, the milk collection container has the two functions of generating a baseline under pressure as well as collecting the expressed milk. The milk collection container for example then has a volume greater than 120ml. This is greater than the volume typically needed for milk collection so that an additional volume serves the function of delivering under pressure. Thus, even when all milk has been expressed, the milk collection chamber remains partially compressed and continues to provide a restoring force. If the volume of the compressible chamber container is too low, the pressure Pc may drop too much when the container is filled with milk until a point that the level of underpressure is no longer able to old the device on the breast.

However, even in this case, the user may re-apply the baseline pressure by squeezing the compressible chamber. Thus, depending on the volumes of the parts (and any valve leakage), the breast pump may be designed such that the user is meant to reapply a squeezing pressure at various intervals, or else the initial fitting may be sufficient for the duration of a milk expression session.

In another set of examples, the breast pump further comprises a milk collection container, additional to the compressible chamber. In this case, the compressible chamber is used only to create a mechanical under pressure. The milk collection container is then a rigid container.

There may be a cyclic pressure waveform for stimulation and a separate waveform for expression. There may also be a massage function. Thus, the addition of the manually generated baseline pressure in accordance with the invention does not detract from the range of possible electrically generated waveforms that may be used. A wearable breast pump will have a battery, not shown above.

In the example above, the valves 46 and 48 are passive valves, so they simply open when the correct pressure differential is present. The valves 46, 48 may instead be controllable, e.g. electrically switchable into an open and/or closed state. In this way, the pressure in the compressible chamber 40 and/or the cavity 42 may be dynamically controllable. Pressure sensors may be used to implement a feedback control approach.

For example, the pressure in the compressible chamber 40 may be reduced (i.e. the compression level recharged) when a negative pressure is generated in cavity 42 by opening the valve 46. However, the compressible chamber remains manually actuatable.

The valves may be only actively controlled, or only passive, or they may be passive valves but which can be overridden into a closed and/or open state regardless of the prevailing pressures.

In the example above, the pump for generating the pressure waveform to the cavity is electric. However, the pump may be a mechanical, manually operated, pump. The separate application of the baseline pressure using the compressible chamber assists the holding of the breast shield over the breast when the mechanical pump is not delivering an under pressure, in the same way as for the electric example described above.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump, comprising:
a breast shield (7) for fitting over at least the nipple of a breast thereby to create a cavity over the breast;
a pump (14,16) for delivering an under pressure to the cavity; and
a compressible chamber (40) for manually creating a reduced pressure in the cavity.

2. The breast pump of claim 1, wherein the pump is an electric pump.

3. The breast pump of claim 1 or 2, wherein the compressible chamber (40) is elastic and delivers a restoring force to return to a non-compressed state.

4. The breast pump of any one of claims 1 to 3, wherein the compressible chamber (40) comprises a milk collection container.

5. The breast pump of claim 4, wherein the milk collection container has a volume greater than 120ml.

6. The breast pump of any one of claims 1 to 3, further comprising a milk collection container, additional to the compressible chamber (40).

7. The breast pump of any one of claims 4 to 6, further comprising a first, one-way, valve (46) between the breast shield and the milk collection container.

8. The breast pump of claim 7, wherein the first valve (46) is a duckbill valve.

9. The breast pump of claim 7 or 8, further comprising a second, one-way, valve (48) from the compressible chamber to ambient surroundings.

10. The breast pump of any one of claims 1 to 9, wherein the breast shield comprises a diaphragm (44) between the cavity and the pump.

11. The breast pump of any one of claims 1 to 10, wherein the compressible chamber (40) generates a negative pressure between 2kPa and 10kPa and/or wherein the compressible chamber (40) is formed of a rubber material such as silicone.

12. The breast pump of any one of claims 1 to 11, comprising a wearable breast pump.

13. A method of operating a breast pump for the non-therapeutic expression of milk, comprising:
fitting a breast shield over at least the nipple of a breast thereby to create a cavity over the breast;
manually creating a reduced pressure in the cavity by compressing a compressible chamber;
delivering an under pressure to the cavity using a pump; and
collecting milk in a milk collection container.

14. The method of claim 13, wherein the compressible chamber comprises the milk collection container or the compressible chamber is additional to the milk collection container.

15. The method of claim 13 or 14, comprising delivering the under pressure using an electric pump.
